(19) Europäisches Patentamt / European Patent Office / Office européen des brevets

(11) **EP 4 261 275 B1**

(12) **EUROPEAN PATENT SPECIFICATION**

(45) Date of publication and mention of the grant of the patent:
**25.03.2026 Bulletin 2026/13**

(21) Application number: **21898216.3**

(22) Date of filing: **19.01.2021**

(51) International Patent Classification (IPC):
*C12N 5/071* (2010.01)    *A61L 27/60* (2006.01)
*A61L 27/38* (2006.01)    *A61L 27/36* (2006.01)
*C12N 5/077* (2010.01)

(52) Cooperative Patent Classification (CPC):
**A61L 27/60; A61L 27/20; A61L 27/36;
A61L 27/3633; A61L 27/38; A61L 27/3804;
A61L 27/3813; A61L 27/3886; A61L 27/3891;
A61L 27/52; C12N 5/0656; C12N 5/0698;**
C12N 2533/54; C12N 2533/74; C12N 2533/90

(86) International application number:
**PCT/KR2021/000716**

(87) International publication number:
**WO 2022/114386 (02.06.2022 Gazette 2022/22)**

(54) **ARTIFICIAL SKIN HAVING STRUCTURE AND PROPERTIES BY AGE, AND METHOD FOR PRODUCING SAME**

KÜNSTLICHE HAUT MIT STRUKTUR UND EIGENSCHAFTEN NACH ALTER UND VERFAHREN ZUR HERSTELLUNG DAVON

PEAU ARTIFICIELLE AYANT UNE STRUCTURE ET DES PROPRIÉTÉS EN FONCTION DE L'ÂGE, ET PROCÉDÉ POUR LA PRODUIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2020 KR 20200163297**

(43) Date of publication of application:
**18.10.2023 Bulletin 2023/42**

(73) Proprietor: **Foundation Of Soongsil University-Industry Cooperation**
Seoul 06978 (KR)

(72) Inventors:
 • **JEONG, Jae Hyun**
 Seoul 03695 (KR)
 • **KIM, Yun Gon**
 Seoul 03302 (KR)
 • **LIM, Jun Woo**
 Incheon 21955 (KR)
 • **SHIN, Sung Gyu**
 Busan 47853 (KR)
 • **HAN, Sa Ra**
 Seoul 07029 (KR)
 • **CHO, Sung Woo**
 Bucheon-si, Gyeonggi-do 14472 (KR)

(74) Representative: **Maiwald GmbH**
**Engineering**
**Elisenhof**
**Elisenstrasse 3**
**80335 München (DE)**

(56) References cited:
KR-A- 20190 003 060    KR-A- 20190 003 061
KR-A- 20200 031 018    KR-B1- 102 118 807
US-A1- 2018 112 188

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **NEWTON V. L. ET AL: "Novel approaches to characterize age-related remodelling of the dermal-epidermal junction in 2D, 3D and in vivo", vol. 23, no. 2, 9 August 2016 (2016-08-09), DK, pages 131 - 148, XP093192840, ISSN: 0909-752X, Retrieved from the Internet <URL:https://api.wiley.com/onlinelibrary/tdm/v1/articles/10.1111%2Fsrt.12312> [retrieved on 20240806], DOI: 10.1111/srt.12312**
- **LANGTON ABIGAIL K.; HALAI POONAM; GRIFFITHS CHRISTOPHER E.M.; SHERRATT MICHAEL J.; WATSON RACHEL E.B.: "The impact of intrinsic ageing on the protein composition of the dermal-epidermal junction", MECHANISMS OF AGEING AND DEVELOPMENT., ELSEVIER SEQUOIA, LAUSANNE,, CH, vol. 156, 21 March 2016 (2016-03-21), CH , pages 14 - 16, XP029569563, ISSN: 0047-6374, DOI: 10.1016/j.mad.2016.03.006**

## Description

[Technical Field]

The present invention relates to artificial skin having structure and properties by age, and more specifically to artificial skin having structure and properties, for which effective analysis and application are possible on the basis of age of the skin, as the human skin can be replicated according to age by simple control in the internal structure of properties such as height and width, and a method for manufacturing the same.

[Background Art]

Currently, there is a trend toward banning animal testing worldwide, and artificial skin is a key technology that can replace existing animal testing and lead the market for customized pharmaceutical compositions and cosmetics.

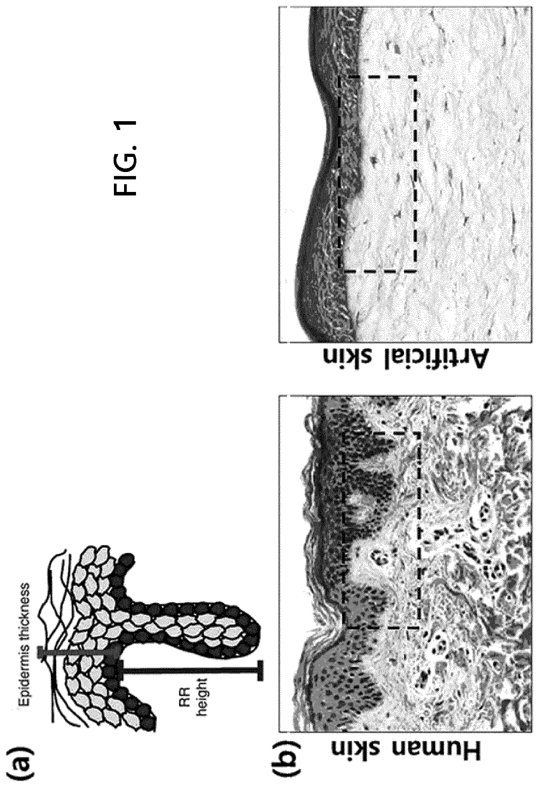

As illustrated in FIGS. 1a and 1b, the dermal-epidermal junction (DEJ) is a junction between the dermis and the epidermis, and it forms a bonding such that the two layers are well-attached. Actual human skin has a structure of rete ridges (concave-convex structure), which not only serves as a mechanical support but also helps the proliferation and differentiation of epidermal cells, facilitates the movement of metabolites and enhances the adhesion between the dermis and epidermis. However, currently, artificial skin is manufactured without this structure.

As such, the related art has problems in that it is a flat artificial skin that does not simulate the concave-convex structure of the dermal-epidermal junction like real skin, and it is manufactured such that the mechanical properties do not differ according to age. It is difficult for the human skin models used in the existing animal replacement test method to have the same characteristics for each manufacturing company, and age-specific simulations have not been performed.

Since human cells have different behaviors depending on the type and physical properties of applied substrates, it is important to model human skin models by age through controlling the physical properties of the dermis layer. In addition, since the structure of human skin varies according to age, the behavior of the epidermis layer also varies according to the structure. Therefore, it is necessary to develop age-specific artificial skin with controlled physical properties and structure for cosmetics research, where formulations and active ingredients vary according to age.

In addition, if artificial skin that simulates the structure of simple skin is referred to as the first generation and artificial skin that implements a full-layer skin model is referred to as the second generation, it is necessary to develop a third-generation artificial skin that can be effectively analyzed and applied according to skin age. Since the structure of human skin, particularly, the concave-convex structure of the dermal-epidermal junction (DEJ) flattens as it ages, it is necessary to manufacture artificial skin that simulates the DEJ concave-convex structure.

In addition, depending on the age (the degree of aging), the skin shows a difference in the degree of crosslinking and bonding strength of the extracellular matrix. The extracellular matrix, which provides a physical environment in which cells can attach and grow in the human body, not only plays a structural role as a cell scaffold, but also serves as a very important cell physiological regulator for cell growth, proliferation and differentiation. Therefore, not only structural simulation but also mechanical properties should be controlled.

Accordingly, as the internal structure of properties such as height, width and the like can be controlled very easily and human skin can be simulated by age, the situation is that there is an urgent need to develop artificial skin that can be effectively analyzed and applied according to skin age.

US 2018/112188 A1 relates to a method of preparing a skin substitute, and to a skin substitute which can be used for the treatment of skin disorders. For the manufacture of the skin substitute, fibroblasts are cultured in a fibroblast culture medium, seeded in a collagen matrix, and culturing the same in a fibroblast culture medium, the matrix and the fibroblasts thus cultured forming a dermal substitute. In addition, melanocytes are cultured in a melanocyte culture medium, and keratinocytes are cultured in a keratinocyte culture medium. The latter two cultures are mixed, and this mixture is seeded on the dermal substitute obtained above. Said dermal substitute is seeded in a skin culture medium, thus forming the skin substitute.

[Disclosure]

[Technical Problem]

The present invention has been devised to solve the above-described problems, and an object of the present invention is to provide artificial skin having structure and properties, for which effective analysis and application are possible on the basis of age of the skin, as the human skin can be replicated according to age by simple control in the internal structure of properties such as height and width, and a method for manufacturing the same.

[Technical Solution]

In order to solve the above-described problems, the present invention provides a method for manufacturing artificial skin, including the steps of (1) controlling mechanical properties by introducing ECM derived substances into a dermis layer; (2) culturing until a dermal-epidermal junction derived from epidermal cells is formed on a surface of the dermis layer into which the ECM derived substances have been introduced, by seeding human epidermis cells on the dermis layer into which the ECM derived substances have been introduced; and (3) manufacturing full-thickness artificial skin having structure and properties by age by differentiating epidermal cells to form a stratum corneum on epidermal cells.

The ECM derived substances may include at least one selected from the group consisting of polysaccharide, elastin, chitosan, hyaluronic acid (HA) and collagen that mimic the structure of glycosaminoglycan (GAG).

In addition, the dermis layer includes a concave-convex structure on a surface thereof.

In addition, the average difference in height between peaks and valleys of the concave-convex structure is 100 $\mu$m or more to 200 $\mu$m or less, in the case of artificial skin at the age of 20 years or more to less than 40 years, wherein the average difference in height between peaks and valleys of the concave-convex structure is 50 $\mu$m or more to less than 100 $\mu$m, in the case of artificial skin at the age of 40 years or more to less than 60 years, and wherein the average difference in height between peaks and valleys of the concave-convex structure is less than 50 $\mu$m, in the case of artificial skin at the age of 60 years or more.

In addition, the distance between a valley of the concave-convex structure and other valleys adjacent to the valley is 250 $\mu$m or less.

In addition, the modulus of elasticity may be 20 to 30 kPa, in the case of artificial skin at the age of 20 years or more to less than 40 years, wherein the modulus of elasticity is 10 to 20 kPa, in the case of artificial skin at the age of 40 years or more to less than 60 years, and wherein the modulus of elasticity is 10 kPa or less, in the case of artificial skin at the age of 60 years or more.

In addition, the concave-convex structure of the dermis layer is manufactured in a shape-changing hydrogel mold prepared by at least one method selected from the group consisting of a shape-changing ductility technique, a three-dimensional lithography technique and a 3D printing technique.

In addition, the culturing of step (2) may be performed by submerged culture.

In addition, the epidermal cells in step (3) may be differentiated through at least one selected from the group consisting of air exposure and air-liquid interface culture (ALIC).

In addition, the epidermal cells may include keratinocytes.

Meanwhile, the present invention provides artificial skin having structure and properties by age, including a dermis layer into which ECM derived substances are introduced; an epidermis layer including sequentially stacked stratum corneum and human epidermis cell layers and provided on top of the dermis layer; and a dermal-epidermal junction provided between the dermis layer and the epidermis layer.

According to the present invention, the dermis layer includes a concave-convex structure on a surface thereof, and wherein the epidermal cell layer and the dermal-epidermal junction may have a shape corresponding to the concave-convex structure of the dermis layer.

According to an exemplary embodiment of the present invention, the shape-changing hydrogel mold may include an acrylic compound including a first acrylic compound and a second acrylic compound.

According to an exemplary embodiment of the present invention, the acrylic compound may include the first acrylic compound and the second acrylic compound at a weight ratio of 5:5 to 1:9.

[Advantageous Effects]

The artificial skin having structure and properties by age according to the present invention and the method for manufacturing the same can very easily control the internal structure of properties such as height, width and the like to simulate human skin by age, and accordingly, it has effects that effective analysis and application according to the skin age are possible.

[Description of Drawings]

FIG. 1a is a mimetic diagram showing the concave-convex structure in the skin, and FIG. 1b is a set of images of staining of human skin tissue and an image of conventional staining of artificial skin.

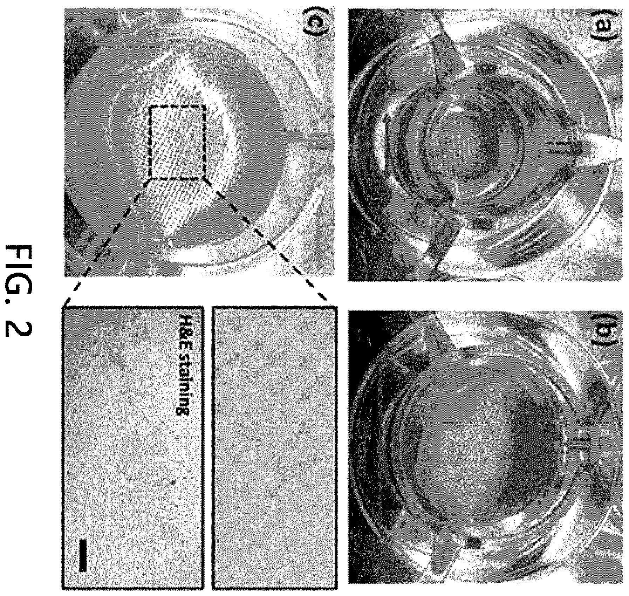

(a) to (c) of FIG. 2 are images showing the concave-convex structure of artificial skin having structure and properties by age according to an exemplary embodiment of the present invention.

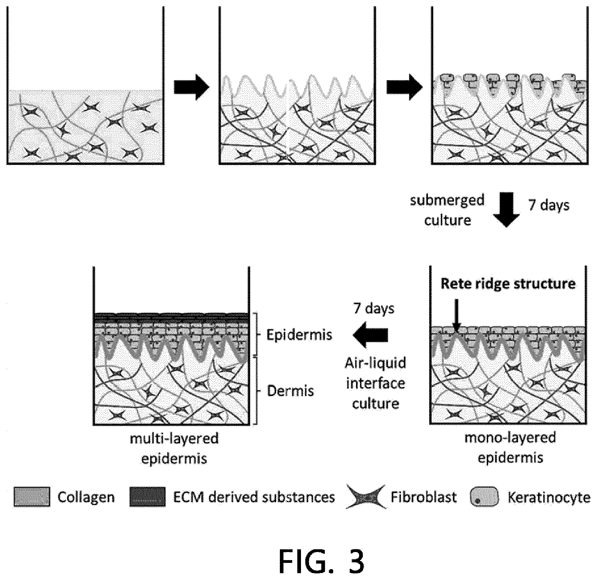

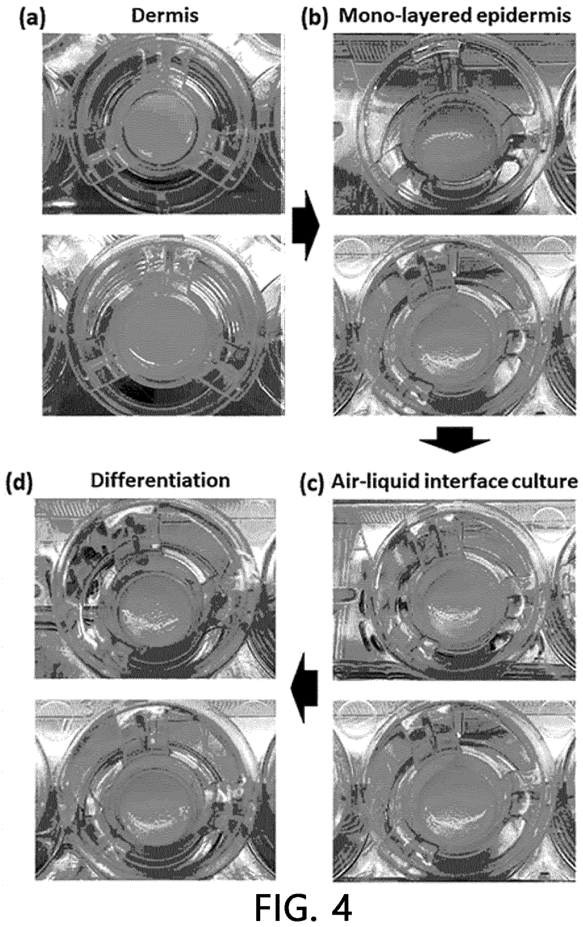

FIGS. 3 and 4 are flowcharts for manufacturing artificial skin having structures and properties by age according to an exemplary embodiment of the present invention.

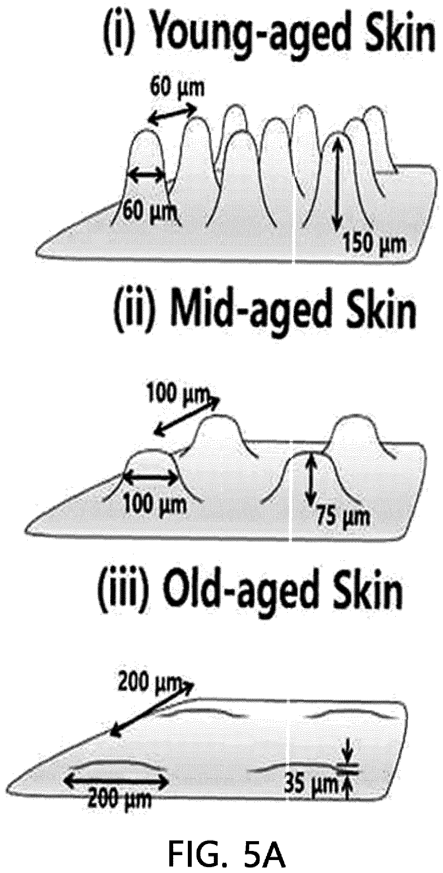

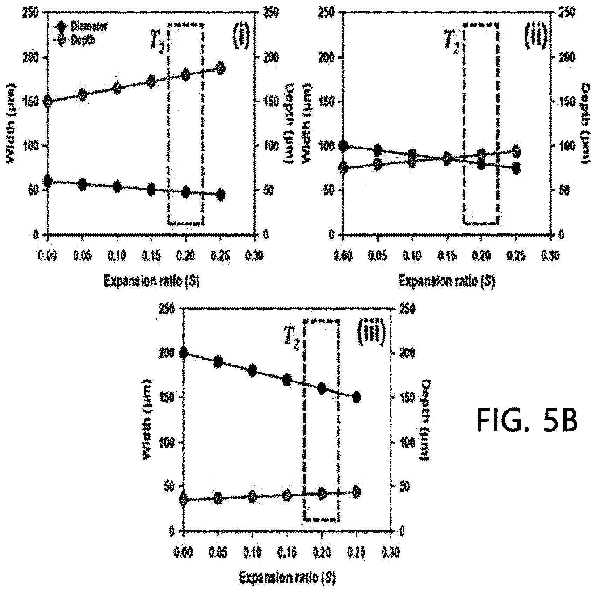

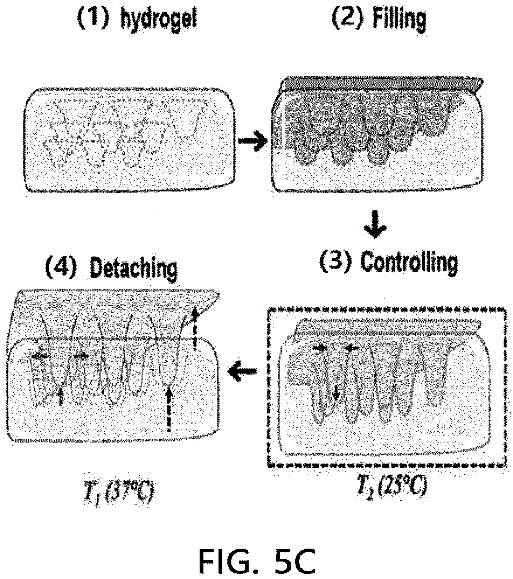

FIG. 5A is a simulation graph according to the diameter, spacing and depth of skin irregularities by age, FIG. 5B is a set of simulation graphs according to the diameter, depth and spacing of skin irregularities by age according to an exemplary embodiment of the present invention, and FIG. 5C is a scheme of the method for manufacturing artificial skin having structure and properties by age according to an exemplary embodiment of the present invention.

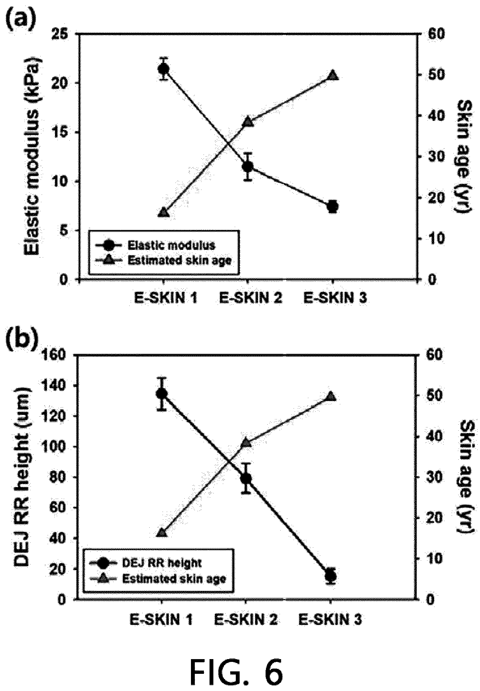

(a) to (b) of FIG. 6 are graphs showing the result values of strength and the concave-convex structure according to the physical properties of artificial skin by age according to an exemplary embodiment of the present invention.

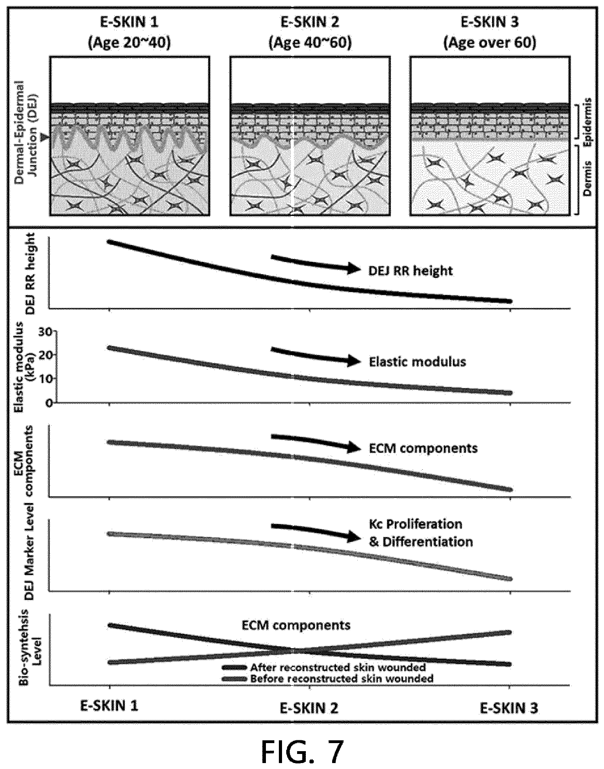

FIG. 7 is a set of graphs showing the structure and physical property behavior of artificial skin having structure and properties by age according to an exemplary embodiment of the present invention.

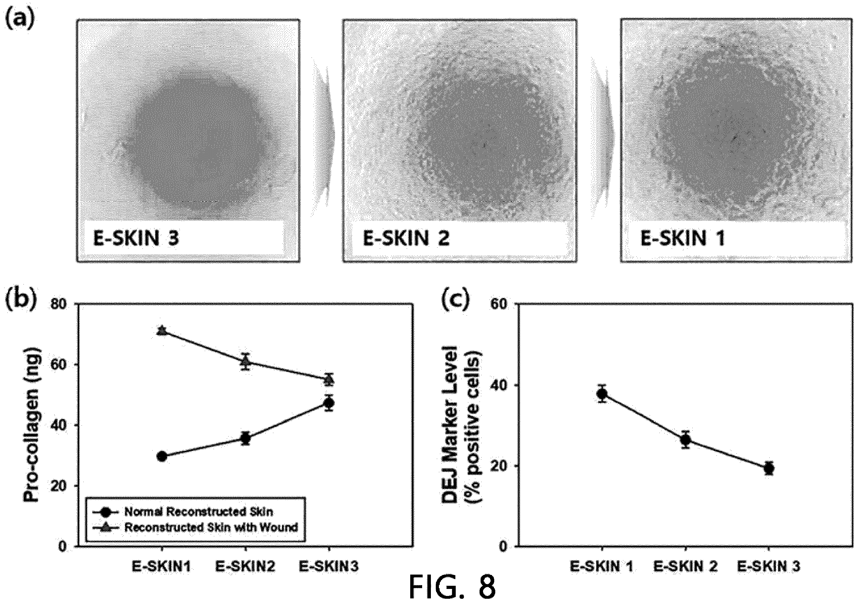

(a) of FIG. 8 is a set of stereoscopic images of artificial skin by age according to an exemplary embodiment of the present invention, (b) of FIG. 8 is a graph showing the amount of pro-collagen expressed before and after wounding on artificial skin having structure and properties by age according to an exemplary embodiment of the present invention, and (c) of FIG. 8 is a graph related to the result values of the DEJ Marker Level.

[Modes of the Invention]

Hereinafter, exemplary embodiments of the present invention will be described in detail so that those of ordinary skill in the art to which the present invention pertains can easily practice the present invention. The present invention may be embodied in many different forms and is not limited to the exemplary embodiments described herein.

As illustrated in FIGS. 3 and 4, artificial skin having structure and physical properties by age according to the present invention may be manufactured by including the steps of (1) controlling mechanical properties by introducing ECM derived substances into a dermis layer; (2) culturing until a dermal-epidermal junction derived from epidermal cells is formed on a surface of the dermis layer into which the ECM derived substances have been introduced, by seeding human epidermis cells on the dermis layer into which the ECM derived substances have been introduced; and (3) manufacturing full-thickness artificial skin having structure and properties by age by differentiating epidermal cells to form a stratum corneum on epidermal cells.

First, step (1) of controlling mechanical properties by introducing ECM derived substances into the dermis layer will be described.

The dermis layer may be used without limitation, as long as it is a material that can be used to form the dermis layer of artificial skin conventionally in the art, and preferably, it may be implemented by including human dermal fibroblasts.

Meanwhile, the ECM derived substances perform a function of controlling the mechanical properties of the artificial skin.

According to age (the degree of aging), the skin shows differences in the degree of crosslinking and bonding strength of the extracellular matrix. The extracellular matrix, which provides a physical environment for cells to attach and grow in the human body, serves not only as a structural support for cells, but also as a very important cell physiology regulator for cell growth, proliferation and differentiation. Therefore, mechanical properties as well as structural simulation must be controlled, which can be achieved through the introduction of ECM derived substances.

The ECM derived substances may be used without limitation, as long as these are substances that can be used to form ECM derived substances that can be introduced into the dermis layer of artificial skin conventionally in the art, and preferably, these substances may include at least one selected from the group consisting of polysaccharide, elastin, chitosan, hyaluronic acid (HA) and collagen that mimic the structure of glycosaminoglycan (GAG), and more preferably, these substances may be implemented by including collagen.

Meanwhile, the ECM derived substances may preferably include collagen, or collagen and methacrylic alginate, and in the case of including the collagen and methacrylic alginate, the collagen and methacrylic alginate may be included at a concentration ratio of 1: 0.2 to 2.0, and preferably, at a concentration ratio of 1: 0.5 to 1.5.

Since the structure of the human skin, particularly the concave-convex structure of the dermis-epidermal junction (DEJ), becomes flatter as it ages, it is necessary to manufacture artificial skin by age in which the structure of the DEJ is simulated.

Accordingly, the dermis layer of the artificial skin having structure and properties by age according to the present invention includes a concave-convex structure on a surface thereof as illustrated in (a) to (c) of FIG. 2.

In addition, the concave-convex structure of the dermis layer is manufactured in a shape-changing hydrogel mold prepared by at least one method selected from the group consisting of ductility and three-dimensional lithography techniques and 3D printing, and preferably, it may be manufactured in a shape-changing hydrogel mold which is capable of controlling the concave-convex structure of the collagen gel of the dermis layer in which the shape changes depending on temperature.

Meanwhile, the shape-changing hydrogel mold may be manufactured through a shape-changing hydrogel mold composition including an acrylic compound including a first acrylic compound and a second acrylic compound.

The acrylic compound constitutes a random copolymer when manufacturing a shape-changing hydrogel mold, and it may include a first acrylic compound and a second acrylic compound as described above.

The first acrylic compound serves as a main structural function of the shape-changing hydrogel mold, and it may be used without limitation as long as it is a first acrylic compound which is polymerizable and has a polymer chain structure, and preferably, it may include at least one selected from the group consisting of acrylamide, poly(ethylene glycol)diacrylate, methacrylated alginate, polyacrylamide, poly(ethylene glycol), methacrylate, sodium polyacrylate, polyacrylate, glyceryl acrylate/acrylic acid copolymer, styrene/acrylate copolymer, sodium acrylate, crosspolymer-2, methyl acrylate, ethyl acrylate, normal butylacrylate and 2-hydroxyethyl methacrylate, and preferably, acrylamide may be used.

In addition, the second acrylic compound functions to express the sensitivity of a shape-changing hydrogel mold, and it may be used without limitation as long as it is a second acrylic compound which is capable of expressing sensitivity to external stimuli in the art, and preferably, it may include at least one selected from the group consisting of N-isopropylacrylamide (NIPAM), acrylic acid, polyvinyl alcohol, poly N-isopropylacrylamide (pNIPAM), poly 2-(dimethylamino)ethyl methacrylate (pDMAEMA), hydroxypropylcellulose (HPC), polyvinylcaprolactame, polyvinyl methyl ether, poly(N,N-dimethyl-lacrylamide), methacrylic acid-methylmethacrylate copolymer, methacrylic acid-methyl acrylate copolymer, methacrylic acid-methylacrylate-methylmethacrylate and poly(2,2-dimethoxy nitrobenzyl methacrylate-r-methyl methacrylate-r-poly(ethylene glycol) methacrylate (PDMP), and preferably, N-isopropyl acrylamide may be used.

In this case, when acrylamide is used as the first acrylic compound and N-isopropyl acrylamide is used as the second acrylic compound, the shape and size of the structure to be manufactured may be easily controlled because the diameter and depth of the mold can be easily controlled, and as the change rate of the diameter and depth of the mold is large, it may be more advantageous to express excellent separability even when manufacturing a structure having low strength.

The acrylic compound may include the first acrylic compound and the second acrylic compound at a weight ratio of 5:5 to 1:9, and preferably, it may include the first acrylic compound and the second acrylic compound at a weight ratio of 3:7 to 1:9. If the weight ratio of the first acrylic compound and the second acrylic compound included in the acrylic compound is less than 5:5, it is not easy to control the diameter and depth of the mold, and thus, it is not easy to control the shape and size when manufacturing a predetermined structure, and there may be a problem in that the change rate of the diameter and depth of the mold becomes small. In addition, if the weight ratio is more than 1:9, there may be a problem in shape control due to rapid contraction and expansion.

Meanwhile, for specific descriptions other than the above-described description of the hydrogel mold, Korean Patent Application No. 10-2018-0147510, which was filed by the same applicant and inventors as the present application, may be incorporated herein by reference, and thus, the detailed description thereof will be omitted.

Meanwhile, when the target age of the artificial skin is 20 years or more to less than 40 years, the average difference in height between peaks and valleys of the concave-convex structure is 100 $\mu$m or more to 200 $\mu$m or less, and preferably, it may be 110 $\mu$m or more to 190 $\mu$m or less. In addition, when the target age of the artificial skin is 40 years or more to less than 60 years, the average difference in height between peaks and valleys of the concave-convex structure is 50 $\mu$m or more to less than 100 $\mu$m, and preferably, it may be 60 $\mu$m or more to 90 $\mu$m or less. In addition, when the target age of the artificial skin is 60 years or more, the average difference in height between peaks and valleys of the concave-convex structure is less than 50 $\mu$m, and preferably, it may be less than 40 $\mu$m.

In addition, the distance between a valley of the concave-convex structure and other valleys adjacent to the valley is 250 $\mu$m or less, and preferably, it may be 220 $\mu$m or less, and by satisfying these, artificial skin by age which is similar to the internal structure of human skin by age may be implemented. As such, when the concave-convex structure is implemented in this way according to age, the same effects and functions as can be seen at the DEJ marker level of FIG. 8 may be exhibited.

Next, step (2) of culturing until the dermal-epidermal junction derived from epidermal cells is formed on the surface of the dermis layer into which the ECM derived substances are introduced, by seeding human epidermis cells on the dermis layer into which the ECM derived substances are introduced will be described.

The epidermal cells may be used without limitation as long as they are materials that can be used as epidermal cells of artificial skin conventionally in the art, and may preferably be implemented by including keratinocytes.

In addition, the culturing in step (2) may be used without limitation as long as it is a method that can be used for cell culture conventionally in the art, and preferably, it may be performed by submerged culture, and more preferably, by submerged culture including DMEM culture media.

Meanwhile, in step (2), the epidermal cells are cultured until the dermal-epidermal junction derived from epidermal cells is formed on the surface of the dermis layer into which the ECM derived substances are introduced. In this case, the dermal-epidermal junction (DEJ) may serve not only as a mechanical support, but also help the proliferation and differentiation of the epidermal cells, facilitate the movement of metabolites and improve the adhesion between the dermis and the epidermis.

Next, step (3) of manufacturing artificial skin having structure and properties by age by differentiating epidermal cells to form a stratum corneum on the epidermal cells will be described.

In step (3), the epidermal cells may be differentiated through any one or more methods selected from air exposure and air-liquid interface culture (ALIC), and preferably, it may be differentiated through air-liquid interface culture (ALIC) using a medium inducing the differentiation of epidermal cells including a certain amount of $Ca^{2+}$.

By forming a stratum corneum on the epidermal cell layer through the differentiation, artificial skin having structure and properties by age may be finally implemented.

Meanwhile, when the target age of the artificial skin is 20 years or more to less than 40 years, the elastic modulus of the artificial skin is 20 to 30 kPa, and preferably, 22 to 28 kPa. In addition, when the target age of the artificial skin is 40 years or more to less than 60 years, the elastic modulus of the artificial skin is 10 to 20 kPa, and preferably, 12 to 18 kPa. In addition, when the target age of the artificial skin is 60 years or more, the elastic modulus of the artificial skin is 10 kPa or less, and preferably, 8 kPa or less. By satisfying these ranges, it is possible to implement artificial skin by age similar to the physical properties of actual human skin by age.

The artificial skin having structure and properties by age according to the present invention may be implemented by including: a dermis layer into which ECM derived substances are introduced; an epidermis layer including sequentially stacked stratum corneum and human epidermis cell layers and provided on top of the dermis layer; and a dermal-epidermal junction provided between the dermis layer and the epidermis layer.

In this case, as described above, the dermis layer may include a concave-convex structure on a surface thereof, and the epidermal cell layer and the dermal-epidermal junction may have a shape corresponding to the concave-convex structure of the dermis layer.

The artificial skin having structure and properties by age according to the present invention and the method for manufacturing the same can very easily control the internal structure of properties such as height and width to simulate human skin by age, and accordingly, it has effects that effective analysis and application according to the skin age are possible.

The present invention will be described in more detail through the following examples, but the following examples are not intended to limit the scope of the present invention, which should be construed to aid understanding of the present invention.

[Example]

### <Preparation Example 1 to Preparation Example 3: Preparation of shape-changing hydrogel mold>

First, a shape-changing hydrogel mold was prepared by radical polymerization reaction through a shape-changing hydrogel mold composition including an acrylic compound including acrylamide as a first acrylic compound and N-isopropyl acrylamide as a second acrylic compound at a weight ratio of 2:8, 0.1 parts by weight of N,N'-methylenebisacrylamide as a crosslinking agent, 1 part by weight of ammonium persulfate as an initiator, 0.1 parts by weight of tetramethylethylenediamine as a catalyst and 88.8 parts by weight of water as a solvent, based on 100 parts by weight of the acrylic compound.

In this case, the shape-changing hydrogel mold was respectively prepared in Preparation Examples 1 to 3 according to the concave-convex structure of artificial skin by age.

In this case, Preparation Example 1 was prepared to have holes having a diameter and interval of 100 $\mu$m or more to 200 $\mu$m or less, and an average difference in height between peaks and valleys of the concave-convex structure of 150 $\mu$m.

In addition, Preparation Example 2 was prepared to have holes having a diameter and interval of 50 $\mu$m or more to 100 $\mu$m or less, and an average difference in height between peaks and valleys of the concave-convex structure of 50 $\mu$m.

In addition, Preparation Example 3 was prepared to have holes having a diameter and interval of less than 0 to less than 50 $\mu$m, and an average difference in height between peaks and valleys of the concave-convex structure of 0 $\mu$m.

### <Experimental Example 1: Simulation of shape-changing hydrogel mold>

The shape-changing hydrogel molds prepared in Preparation Examples 1 to 3 were simulated and evaluated for the degree of change in the average diameter and average depth of the surface according to the expansion ratio through FIGS. 5a and 5b and Relationship Formulas 1 and 2 below.

[Relationship Formula 1]

$$R = R_i - (R_v - 2R_i) \times S$$

In Relationship Formula 1, R is the changed radius of the concave-convex structure, $R_i$ is the radius of the concave-convex structure immediately after being manufactured, $R_v$ is the length from the center of the concave-convex structure to the nearest concave-convex structure, and S is the expansion ratio of the hydrogel.

[Relationship Formula 2]

$$D = D_i + D_i \times S$$

Herein, D is the changed depth of the concave-convex structure, $D_i$ is the depth of the concave-convex structure immediately after being manufactured, and S is the expansion ratio of the hydrogel.

When the operating temperature of the shape-changing mold increased, the bulk gel contracted while the radius of the concave-convex structure increased and the depth decreased. Conversely, when the operating temperature decreased, the gel expanded while the radius of the concave-convex structure decreased and the depth increased.

In spite of the relatively low physical properties of the dermis layer, not only can the depth be automatically controlled, but also after the dermis layer with the controlled concave-convex structure was prepared, the depth of the mold structure was finally lowered, and thus, the dermis layer was naturally separated from the mold without external force.

### <Example 1: Manufacture of artificial skin at age of 20 years or more to less than 40 years, E-SKIN 1>

An artificial skin having structure and physical properties by age was manufactured through the method shown in FIG. 3.

Specifically, first, the dermis layer was formed by using human dermal fibroblasts as the dermis layer-forming cells, the concave-convex structure was introduced, and collagen was introduced as the ECM derived substances.

In addition, as the ECM derived substances to control the strength, methacrylic alginate (MA) in which a methacrylate functional group was introduced into alginate was applied, and it was used at 4.8 mg/mL based on the concentration of collagen at 4.8 mg/mL.

In this case, the mechanical strength was manufactured to be formed to be 25 ($\pm$5) kPa, and for the introduction of the concave-convex structure, in order to increase the roughness, the mold prepared in Preparation Example 1 above having holes with a diameter and interval of 100 to 200 $\mu$m and a depth of 100 $\mu$m was used when the dermis layer was manufactured (FIG. 5C).

In addition, it was cultured by submerged culture until the dermal-epidermal junction derived from epidermal cells was formed on the surface of the dermis layer by seeding keratinocytes with human epidermis cells on the dermis layer into which the concave-convex structure was introduced.

Thereafter, epidermal cells were differentiated through air-liquid interface culture (ALIC) to form a stratum corneum on epidermal cells, and finally, artificial skin having structure and properties by age, which included a dermis layer into which ECM derived substances were introduced; an epidermis layer including sequentially stacked stratum corneum and human epidermis cell layers and provided on top of the dermis layer; and a dermal-epidermal junction provided between the dermis layer and the epidermis layer, was manufactured.

### <Example 2: Manufacture of artificial skin at age of 40 years or more to less than 60 years, E-SKIN 2>

Artificial skin was manufactured in the same manner as in Example 1, except that methacrylic alginate (MA) was used at 2.4 mg/mL based on collagen at a concentration of 4.8 mg/mL, and the mechanical strength was manufactured to be formed at a concentration of 15 ($\pm$5) kPa, and Example 2 was carried out by using the mold prepared in Preparation Example 2.

### <Example 3: Manufacture of artificial skin at age of 60 years or more, E-SKIN 3>

Artificial skin was manufactured in the same manner as in Example 1, except that methacrylic alginate (MA) was used at 0 mg/mL based on collagen at a concentration of 4.8 mg/mL, and the mechanical strength was manufactured to be formed at a concentration of 5 ($\pm$5) kPa, and Example 3 was carried out by using the mold prepared in Preparation Example 3.

### <Experimental Example 2: Evaluation of the concave-convex structure of artificial skin having structure and properties by age>

The concave-convex structure of the artificial skin was evaluated for the artificial skin having structure and properties by age manufactured according to Example 1.

As a result, as illustrated in FIG. 2, when viewed with the naked eye, it looked like small dots, and when it was magnified with a stereoscope and an optical microscope, it was confirmed that the rete ridge structure was formed and well-maintained. In addition, when the cross-section was viewed through H&E staining, it was confirmed that the height and width were properly formed according to the standard designed by the rete ridge structure. In addition, it was confirmed that the weak structure of collagen was maintained without collapsing even after culturing for 14 days.

In addition, as shown in (a) to (b) of FIG. 6, it was confirmed that the physical properties having the strength and concave-convex structure for each characteristic age were secured.

In addition, (a) of FIG. 8 is an image obtained by photographing Example 1 (E-SKIN 1), Example 2 (E-SKIN 2) and Example 3 (E-SKIN 3) with a stereoscope, and compared to E-SKIN 1, it was confirmed that E-SKIN 3 had relatively flat skin and did not have many irregularities.

### <Experimental Example 3: Evaluation of age-specific structure and physical property behaviors of artificial skin having structure and properties by age>

For the artificial skins having structure and properties by age manufactured according to Examples 1 to 3, the structure and physical property behavior by age were evaluated.

As a result, as illustrated in FIG. 7, as Example 3 simulated relatively old skin, the average difference in height between peaks and valleys of the concave-convex structure of the dermal-epidermal junction was lowered, and the amount of the ECM derived substances decreased, and accordingly, it can be confirmed that the strength (the modulus of elasticity) decreased as the amount of the ECM derived substances decreased.

In addition, as the concave-convex structure of the dermal-epidermal junction helps the proliferation and differentiation of epidermal cells, it can be confirmed that the degree of proliferation and differentiation of epidermal cells decreased toward Example 3 simulating relatively old skin.

In addition, when the biosynthetic amounts (procollagen) of the labeling materials in each of the artificial skins having structure and properties by age according to Examples 1 to 3 were compared, as shown in (b) of FIG. 8, it can be confirmed that the synthetic amount was low in Example 1, and the synthetic amount was high in Example 3. This is a phenomenon that occurs because Example 3 had a small amount of extracellular matrix compared to Example 1. However, it can be seen that when a wound was made, the cell behavior according to the age was exhibited well, as Example 1 showed a greater synthetic amount compared to Example 3. Since this coincides with better recovery in actual young skin, it can be seen that artificial skins by age had been successfully implemented.

In addition, as can be seen from (c) of FIG. 8, when the DEJ marker level was further confirmed, it can be confirmed that the expression amount was high in Example 1 where the concave-convex structure was well-formed.

**<Experimental Example 4: Evaluation of the amount of pro-collagen type I expressed in artificial skin having structure and properties by age>**

The amount of pro-collagen type I expressed before and after wounding was evaluated for the artificial skins having structure and properties by age manufactured according to Examples 1 to 3.

As a result, it can be confirmed that the expression level of pro-collagen type I after manufacturing artificial skins implemented by age was higher in Example 3 (E-SKIN3) with less extracellular matrix, that is, in artificial skin implemented with a higher age. Since there was no extracellular matrix, it can be considered that the active metabolism of fibroblasts to create the same had occurred. However, it was confirmed that the expression of pro-collagen type I in Example 1 (E-SKIN1), that is, young skin, was significantly increased when the artificial skins by age were wounded. Conversely, in the case of Example 3 (E-SKIN3) which is old skin, there was not much difference in the amounts of expression after manufacturing. Therefore, as in real skin, it can be demonstrated by the expression level of pro-collagen type I that the replication of young skin had better recovery than the replication of old skin among the artificial skins by age.

**Claims**

1. A method for manufacturing artificial skin, comprising the steps of:

    (1) controlling mechanical properties by introducing ECM derived substances into a dermis layer;
    (2) culturing until a dermal-epidermal junction derived from epidermal cells is formed on a surface of the dermis layer into which the ECM derived substances have been introduced, by seeding human epidermis cells on the dermis layer into which the ECM derived substances have been introduced; and
    (3) manufacturing full-thickness artificial skin having structure and properties by age by differentiating epidermal cells to form a stratum corneum on epidermal cells,

    wherein the dermis layer comprises a concave-convex structure on a surface thereof,
    wherein the concave-convex structure of the dermis layer is manufactured in a shape-changing hydrogel mold prepared by at least one method selected from the group consisting of a shape-changing ductility technique, a three-dimensional lithography technique and a 3D printing technique,
    wherein the shape-changing hydrogel mold changes shape depending on temperature to control the concave-convex structure of the dermis layer,
    wherein the distance between a valley of the concave-convex structure and other valleys adjacent to the valley is 250 $\mu$m or less,
    wherein the average difference in height between peaks and valleys of the concave-convex structure is 100 $\mu$m or more to 200 $\mu$m or less, and wherein the modulus of elasticity is 20 to 30 kPa, when the target age of the artificial skin is 20 years or more to less than 40 years,
    wherein the average difference in height between peaks and valleys of the concave-convex structure is 50 $\mu$m or more to less than 100 $\mu$m, and wherein the modulus of elasticity is 10 to 20 kPa, when the target age of the artificial skin is 40 years or more to less than 60 years, and
    wherein the average difference in height between peaks and valleys of the concave-convex structure is less than 50 $\mu$m, and wherein the modulus of elasticity is 10 kPa or less, when the target age of the artificial skin is 60 years or more.

2. The method of claim 1, wherein the dermis layer comprises human dermal fibroblasts, and
    wherein the ECM derived substances comprise at least one selected from the group consisting of polysaccharide, elastin, chitosan, hyaluronic acid (HA) and collagen that mimic the structure of glycosaminoglycan (GAG).

3. The method of claim 1, wherein the culturing of step (2) is performed by submerged culture.

4. The method of claim 1, wherein the epidermal cells in step (3) are differentiated through at least one selected from the group consisting of air exposure and air-liquid interface culture (ALIC).

5. The method of claim 1, wherein the epidermal cells comprise keratinocytes.

6. Artificial skin having structure and properties by age, comprising:

a dermis layer into which ECM derived substances are introduced;
an epidermis layer comprising sequentially stacked stratum corneum and human epidermis cell layers and provided on top of the dermis layer; and
a dermal-epidermal junction provided between the dermis layer and the epidermis layer,
wherein the dermis layer comprises a concave-convex structure on a surface thereof,
wherein the epidermal cell layer and the dermal-epidermal junction have a shape corresponding to the concave-convex structure of the dermis layer,
wherein the distance between a valley of the concave-convex structure and other valleys adjacent to the valley is 250 μm or less,
wherein the average difference in height between peaks and valleys of the concave-convex structure is 100 μm or more to 200 μm or less, and wherein the modulus of elasticity is 20 to 30 kPa, when the target age of the artificial skin is 20 years or more to less than 40 years,
wherein the average difference in height between peaks and valleys of the concave-convex structure is 50 μm or more to less than 100 μm, and wherein the modulus of elasticity is 10 to 20 kPa, when the target age of the artificial skin is 40 years or more to less than 60 years, and
wherein the average difference in height between peaks and valleys of the concave-convex structure is less than 50 μm, and wherein the modulus of elasticity is 10 kPa or less, when the target age of the artificial skin is 60 years or more.

7. The method of claim 1, wherein the shape-changing hydrogel mold comprises an acrylic compound comprising a first acrylic compound and a second acrylic compound.

8. The method of claim 7, wherein the acrylic compound comprises the first acrylic compound and the second acrylic compound at a weight ratio of 5:5 to 1:9.

**Patentansprüche**

1. Verfahren zur Herstellung künstlicher Haut, umfassend die Schritte:

(1) Steuern der mechanischen Eigenschaften durch Einbringen von aus der ECM gewonnenen Substanzen in eine Dermisschicht;
(2) Kultivieren, bis sich eine von epidermalen Zellen stammende dermoepidermale Junktionszone auf einer Oberfläche der Dermisschicht bildet, in welche aus der ECM gewonnenen Substanzen eingebracht wurden, indem menschliche epidermale Zellen auf die Dermisschicht ausgesät werden, in welche aus der ECM gewonnenen Substanzen eingebracht wurden; und
(3) Herstellen einer vollschichtigen künstlichen Haut mit altersabhängiger Struktur und altersabhängigen Eigenschaften durch Differenzieren epidermaler Zellen zur Ausbildung eines Stratum corneum auf epidermalen Zellen,
wobei die Dermisschicht eine konkav-konvexe Struktur auf ihrer Oberfläche aufweist,
wobei die konkav-konvexe Struktur der Dermisschicht in einer formverändernden Hydrogelform hergestellt wird, die durch mindestens ein Verfahren ausgewählt aus der Gruppe bestehend aus einer formveränderlichen Duktilitätstechnik, einer dreidimensionalen Lithographietechnik und einer 3D-Drucktechnik, vorbereitet wurde,
wobei die formverändernde Hydrogelform ihre Form in Abhängigkeit von der Temperatur verändert, um die konkav-konvexe Struktur der Dermisschicht zu steuern,
wobei der Abstand zwischen einer Vertiefung der konkav-konvexen Struktur und anderen, an diese Vertiefung angrenzenden Vertiefungen 250 μm oder weniger beträgt,
wobei die durchschnittliche Höhendifferenz zwischen Erhebungen und Vertiefungen der konkav-konvexen Struktur 100 μm oder mehr bis 200 μm oder weniger beträgt und wobei der Elastizitätsmodul 20 bis 30 kPa beträgt, wenn das Zielalter der künstlichen Haut 20 Jahre oder mehr bis weniger als 40 Jahre beträgt,
wobei die durchschnittliche Höhendifferenz zwischen Erhebungen und Vertiefungen der konkav-konvexen Struktur 50 μm oder mehr bis weniger als 100 μm beträgt und wobei der Elastizitätsmodul 10 bis 20 kPa beträgt, wenn das Zielalter der künstlichen Haut 40 Jahre oder mehr bis weniger als 60 Jahre beträgt, und
wobei die durchschnittliche Höhendifferenz zwischen Erhebungen und Vertiefungen der konkav-konvexen Struktur weniger als 50 μm beträgt und wobei der Elastizitätsmodul 10 kPa oder weniger beträgt, wenn das Zielalter der künstlichen Haut 60 Jahre oder mehr beträgt.

2. Verfahren nach Anspruch 1, wobei die Dermisschicht menschliche dermale Fibroblasten umfasst, und

wobei die aus der ECM gewonnenen Substanzen mindestens eine ausgewählt aus der Gruppe bestehend aus Polysaccharid, Elastin, Chitosan, Hyaluronsäure (HA) und Kollagen umfassen, die die Struktur von Glykosaminoglykan (GAG) nachahmen.

3. Verfahren nach Anspruch 1, wobei die Kultivierung in Schritt (2) als Submerskultur durchgeführt wird.

4. Verfahren nach Anspruch 1, wobei die epidermalen Zellen in Schritt (3) durch mindestens eine aus der Gruppe bestehend aus Luftexposition und Luft-Flüssigkeits-Grenzflächenkultur (ALIC) differenziert werden.

5. Verfahren nach Anspruch 1, wobei die epidermalen Zellen Keratinozyten umfassen.

6. Künstliche Haut mit altersabhängiger Struktur und altersabhängigen Eigenschaften, umfassend:

eine Dermisschicht, in die aus der ECM gewonnenen Substanzen eingebracht werden;
eine Epidermisschicht, die sequenziell angeordnete Stratum-corneum- und menschliche Epidermiszellschichten umfasst und auf der Dermisschicht angeordnet ist; und
eine dermoepidermale Junktionszone, die zwischen der Dermisschicht und der Epidermisschicht gebildet ist,
wobei die Dermisschicht eine konkav-konvexe Struktur auf ihrer Oberfläche aufweist,
wobei die epidermale Zellschicht und die dermoepidermale Junktionszone eine Form aufweisen, die der konkav-konvexen Struktur der Dermisschicht entspricht,
wobei der Abstand zwischen einer Vertiefung der konkav-konvexen Struktur und anderen, an diese Vertiefung angrenzenden Vertiefungen 250 $\mu$m oder weniger beträgt,
wobei die durchschnittliche Höhendifferenz zwischen Erhebungen und Vertiefungen der konkav-konvexen Struktur 100 $\mu$m oder mehr bis 200 $\mu$m oder weniger beträgt und wobei der Elastizitätsmodul 20 bis 30 kPa beträgt, wenn das Zielalter der künstlichen Haut 20 Jahre oder mehr bis weniger als 40 Jahre beträgt,
wobei die durchschnittliche Höhendifferenz zwischen Erhebungen und Vertiefungen der konkav-konvexen Struktur 50 $\mu$m oder mehr bis weniger als 100 $\mu$m beträgt und wobei der Elastizitätsmodul 10 bis 20 kPa beträgt, wenn das Zielalter der künstlichen Haut 40 Jahre oder mehr bis weniger als 60 Jahre beträgt, und
wobei die durchschnittliche Höhendifferenz zwischen Erhebungen und Vertiefungen der konkav-konvexen Struktur weniger als 50 $\mu$m beträgt und wobei der Elastizitätsmodul 10 kPa oder weniger beträgt, wenn das Zielalter der künstlichen Haut 60 Jahre oder mehr beträgt.

7. Verfahren nach Anspruch 1, wobei die formverändernde Hydrogelform eine Acrylverbindung umfasst, die eine erste Acrylverbindung und eine zweite Acrylverbindung umfasst.

8. Verfahren nach Anspruch 7, wobei die Acrylverbindung die erste Acrylverbindung und die zweite Acrylverbindung in einem Gewichtsverhältnis von 5:5 bis 1:9 umfasst.

**Revendications**

1. Procédé de fabrication de peau artificielle, comprenant les étapes consistant à :

(1) contrôler des propriétés mécaniques en introduisant des substances dérivées de la MEC dans une couche dermique ;
(2) mettre en culture jusqu'à ce qu'une jonction dermo-épidermique dérivée de cellules épidermiques soit formée sur une surface de la couche dermique dans laquelle les substances dérivées de la MEC ont été introduites, en ensemençant des cellules d'épiderme humain sur la couche dermique dans laquelle les substances dérivées de la MEC ont été introduites ; et
(3) fabriquer de la peau artificielle d'épaisseur totale présentant une structure et des propriétés en fonction de l'âge en différenciant des cellules épidermiques pour former une couche cornée sur des cellules épidermiques,
dans lequel la couche dermique comprend une structure concave-convexe sur une surface de celle-ci,
dans lequel la structure concave-convexe de la couche dermique est fabriquée dans un moule en hydrogel à changement de forme préparé selon au moins un procédé sélectionné dans le groupe constitué d'une technique de ductilité à changement de forme, une technique de lithographie tridimensionnelle, et une technique d'impression 3D,
dans lequel le moule en hydrogel à changement de forme change de forme en fonction de la température pour contrôler la structure concave-convexe de la couche dermique,
dans lequel la distance entre un creux de la structure concave-convexe et d'autres creux adjacents au creux est inférieure ou égale à 250 $\mu$m,
dans lequel la différence moyenne de hauteur entre des sommets et des creux de la structure concave-convexe est supérieure ou égale à 100 μm et inférieure ou égale à 200 μm et dans lequel le module d'élasticité est compris entre 20 et 30 kPa quand l'âge cible de la peau artificielle est supérieur ou égal à 20 ans et inférieur à 40 ans,

dans lequel la différence moyenne de hauteur entre des sommets et des creux de la structure concave-convexe est supérieure ou égale à 50 μm et inférieure à 100 μm et dans lequel le module d'élasticité est compris entre 10 et 20 kPa quand l'âge cible de la peau artificielle est supérieur ou égal à 40 ans et inférieur à 60 ans, et

dans lequel la différence moyenne de hauteur entre des sommets et des creux de la structure concave-convexe est inférieure à 50 μm et dans lequel le module d'élasticité est inférieur ou égal à 10 kPa quand l'âge cible de la peau artificielle est supérieur ou égal à 60 ans.

2. Procédé selon la revendication 1, dans lequel la couche dermique comprend des fibroblastes dermiques humains, et

dans lequel les substances dérivées de la MEC comprennent au moins un élément sélectionné dans le groupe constitué du polysaccharide, de l'élastine, du chitosane, de l'acide hyaluronique (AH), et du collagène qui imitent la structure du glycosaminoglycane (GAG).

3. Procédé selon la revendication 1, dans lequel la mise en culture de l'étape (2) est effectuée par culture immergée.

4. Procédé selon la revendication 1, dans lequel les cellules épidermiques de l'étape (3) sont différenciées par au moins une méthode sélectionnée dans le groupe constitué de l'exposition à l'air et la culture à l'interface air-liquide (CIAL).

5. Procédé selon la revendication 1, dans lequel les cellules épidermiques comprennent des kératinocytes.

6. Peau artificielle présentant une structure et des propriétés en fonction de l'âge, comprenant :

une couche dermique dans laquelle des substances dérivées de la MEC sont introduites ;
une couche épidermique comprenant des couches de cellules épidermiques humaines et de couche cornée empilées de manière séquentielle et située sur la couche dermique ; et
une jonction dermo-épidermique située entre la couche dermique et la couche épidermique,
dans laquelle la couche dermique comprend une structure concave-convexe sur une surface de celle-ci,

dans laquelle la couche de cellules épidermiques et la jonction dermo-épidermique présentent une forme correspondant à la structure concave-convexe de la couche dermique,
dans laquelle la distance entre un creux de la structure concave-convexe et d'autres creux adjacents au creux est inférieure ou égale à 250 μm,
dans laquelle la différence moyenne de hauteur entre des sommets et des creux de la structure concave-convexe est supérieure ou égale à 100 μm et inférieure ou égale à 200 μm et dans lequel le module d'élasticité est compris entre 20 et 30 kPa quand l'âge cible de la peau artificielle est supérieur ou égal à 20 ans et inférieur à 40 ans,
dans laquelle la différence moyenne de hauteur entre des sommets et des creux de la structure concave-convexe est supérieure ou égale à 50 μm et inférieure à 100 μm et dans lequel le module d'élasticité est compris entre 10 et 20 kPa quand l'âge cible de la peau artificielle est supérieur ou égal à 40 ans et inférieur à 60 ans, et
dans laquelle la différence moyenne de hauteur entre des sommets et des creux de la structure concave-convexe est inférieure à 50 μm et dans lequel le module d'élasticité est inférieur ou égal à 10 kPa quand l'âge cible de la peau artificielle est supérieur ou égal à 60 ans.

7. Procédé selon la revendication 1, dans lequel le moule en hydrogel à changement de forme comprend un composé acrylique comprenant un premier composé acrylique et un second composé acrylique.

8. Procédé selon la revendication 7, dans lequel le composé acrylique comprend le premier composé acrylique et le second composé acrylique dans un rapport en poids de 5:5 à 1:9.

FIG. 1

(a) Epidermis thickness

RR height

(b) Human skin

Artificial skin

FIG. 2

submerged culture | 7 days

Rete ridge structure

Epidermis

Dermis

7 days

Air-liquid interface culture

multi-layered epidermis

mono-layered epidermis

Collagen ECM derived substances Fibroblast Keratinocyte

FIG. 3

(a) Dermis    (b) Mono-layered epidermis

(d) Differentiation    (c) Air-liquid interface culture

FIG. 4

FIG. 5A

FIG. 5B

**(1) hydrogel**

**(2) Filling**

**(4) Detaching**

**(3) Controlling**

$T_1 (37°C)$

$T_2 (25°C)$

## FIG. 5C

FIG. 6

FIG. 7

FIG. 8

**EP 4 261 275 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018112188 A1

- KR 1020180147510